# EUROPÄISCHE PATENTSCHRIFT

(11) **EP 0 378 203 B1**
(45) Veröffentlichungstag und Bekanntmachung des Hinweises auf die Patenterteilung: **28.08.1996**
(21) Anmeldenummer: 90100462.2
(22) Anmeldetag: 10.01.1990
(51) Int. Cl.: C07H 23/00, G01N 33/82, G01N 33/531

(54) **Neue Cobalamin-Säurehydrazide und davon abgeleitete Cobalamin-Konjugate**
Cobalamine-acid hydrazides and cobalamine conjugates derived therefrom
Acide-hydrazide de la cobalamine et conjugués de la cobalamine en dérivant

(30) Priorität: 11.01.1989 DE 3900648
(43) Veröffentlichungstag der Anmeldung: 18.07.1990
(73) Patentinhaber: BOEHRINGER MANNHEIM GMBH, 68298 Mannheim (DE)
(72) Erfinder: Huber, Erasmus, Dr. rer. nat., D-8046 Garching (DE); Dieckhoff, Josef, Dr. rer. nat., D-8133 Feldafing (DE); Klein, Christian, Dr. rer. nat., D-8120 Weilheim (DE); Kürzinger, Konrad, Dr. rer. nat., D-8132 Tutzing (DE)
(74) Vertreter: Huber, Bernhard, Dipl.-Chem.

(56) Entgegenhaltungen:
- EP-A- 0 069 450
- DE-A- 2 019 176
- US-A- 3 981 863

## Beschreibung

Die Erfindung betrifft neue Cobalamin-Säurehydrazide, insbesondere Cyanocobalamin-Säurehydrazide, und davon abgeleitete Cobalamin-Konjugate.

Die immunologische Bestimmung von Cobalaminen (Vitamin B12) erfolgt üblicherweise in einem kompetitiven Test, bei dem das Cobalamin der Probe und radioaktiv markiertes Cobalamin um ein festphasengebundenes Bindeprotein für Cobalamin (intrinsic factor) konkurrieren (RIA-Test). Nach Trennung der festen von der flüssigen Phase kann in einer der beiden Phasen aus der dort enthaltenen Menge der radioaktiven Markierung die Menge des in der Probe enthaltenen Cobalamins ermittelt werden.

Aufgrund der bekannten Nachteile von RIA-Tests (z.B. radioaktive Belastung, Entsorgung) ist es wünschenswert, die radioaktive Markierung durch eine enzymatische Markierung zu ersetzen.

Bekannte Kupplungstechniken, wie sie z.B. zur Herstellung von Protein-Vitamin B12-Konjugaten als Immunogene beschrieben sind (vgl. H. Gershman et al., Archives of Biochemistry and Biophysics 153, (1972), 407; D.F.M. v. d. Wiel et al., Clin. Chim. Acta 56 (1974), 143; D.B. Endres et al., Clin. Chem. 24/3 (1978), 460; US-Patent 3,981,863), führen nicht zu stabilen B12-Derivaten. Entsprechende Verfahren werden auch zur Herstellung von B12-Enzymkonjugaten beschrieben.

Derartig hergestellte B12-Derivate sind hydrolyseempfindlich und nur als instabile Zwischenstufen formulierbar. Die Kupplungsausbeute ist deshalb relativ niedrig und, wenn überhaupt, nur schwer reproduzierbar. Zudem findet eine beträchtliche Polymerisation des Enzyms als Nebenreaktion statt.

Aufgabe der vorliegenden Erfindung ist es, isolierbare und beständige Cobalamin-Säurederivate bereitzustellen, die diese Nachteile nicht aufweisen und mit denen sich Konjugate auf einfache und reproduzierbare Weise erhalten lassen. Diese Aufgabe wird mit den erfindungsgemäß bereitgestellten neuen Cobalaminsäure-Hydraziden gelöst.

Gegenstand der Erfindung sind Cobalamin-Säurehydrazide gemäß Anspruch 1.

Vorzugsweise ist B der von Cyano-Cobalamin abgeleitete Rest B12, besonders bevorzugt besitzen diese Hydrazide die Formel II 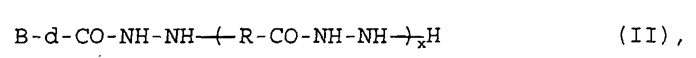 worin B, R und x die angegebene Bedeutung besitzen und d die Stellung von -CO-NH-NH- bedeutet. Bei diesen Verbindungen bedeutet -R-CO- den Rest 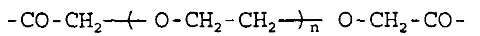 mit n=1, 2 oder 3, insbesondere entsprechen sie der Formel B12-d-CO-NH-NH₂, worin B12 und d die oben angegebene Bedeutung besitzen, oder der Formel 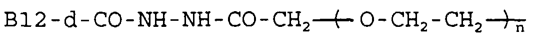 O-CH₂-CO-NH-NH₂, worin B12 und d die oben angegebene Bedeutung besitzen, mit n=1, 2 oder 3.

Erfindungsgemäß werden die aus den Säureamidgruppen -CO-NH₂ der Cobalamine der nachstehenden Formel 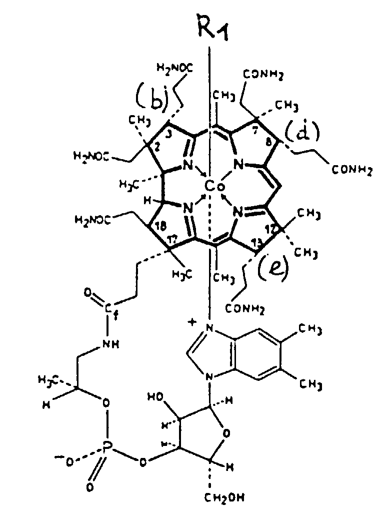 in der R₁ die verschiedenen, die einzelnen Cobalamine unterscheidenden Reste darstellt (für das Cyanocobalamin ist R₁=CN; vgl. z.B. Römpps, Chemie-Lexikon, Band 2, 8. Auflage, 1981, Franckh'sche Verlagshandlung Stuttgart, Seite 784) erhaltenen freien Carboxylgruppen in die Hydrazide der allgemeinen Formel (I) überführt.

Die Überführung der Säureamidgruppe -CO-NH₂ in die freie Carboxylgruppe erfolgt durch saure Verseifung und Isolierung der freien Carbonsäuren (vgl. R. Yamada und H.P.C. Hogenkamp, J. Biol. Chem. 1972 (247) 6266-6270; D.L. Anton et al., J. Amer. Chem. Soc. 102 (1980), 2215). Vorzugsweise arbeitet man so, daß eine unvollständige Verseifung der im Cobalamin-Molekül vorhandenen Säureamidgruppen erreicht wird, damit nur eine oder einige wenige der entsprechenden Carboxylgruppen freigesetzt werden. Aus dem so erhaltenen Gemisch kann die Verbindung isoliert werden, in der die freie Carboxylgruppe an einer bestimmten Stelle vorliegt. Vorzugsweise wird die Verbindung mit der freien Carboxylgruppe in d-Stellung (vgl. die vorstehende Formel der Cobalamine) isoliert. Diese freien Carbonsäuren werden dann durch weitere Umsetzung ins entsprechende Hydrazid der allgemeinen Formel (I) überführt. Es ist auch möglich, von einem Gemisch der Cobalamin-Carbonsäuren auszugehen unter Bildung eines Gemisches der entsprechenden Hydrazide. Zur Herstellung eines CobalaminKonjugates für die Verwendung in Immunoassays ist es aber im Hinblick auf die Empfindlichkeit der Bestimmungsmethode zweckmäßig und deshalb auf alle Fälle vorzuziehen, von einer einzigen gereinigten Carbonsäure auszugehen und diese in das entsprechende reine, definierte Hydrazid überzuführen.

Vorzugsweise werden die Cobalamin-Monocarbonsäuren durch Umsetzung mit einem Carbodiimid, wie z.B. N-Ethyl-N'-(dimethylaminopropyl)carbodiimid, und einer entsprechenden Hydrazinverbindung, in die Carbonsäurehydrazide der Formel (I) übergeführt. Die Herstellung der Verbindungen der allgemeinen Formel (I), worin x=1, kann auch durch einen schrittweisen Aufbau der an B gebundenen Gruppierung erfolgen, z.B. mit der Reaktionsfolge B-COOH → B-CO-NH-NH-R-COOH → B-CO-NH-NH-R-CO-NH-NH₂, oder aber auch durch Umsetzung mit einer Verbindung H₂N-NH-R-CO-NH-NH₂, z.B. mit Triethylenglykol-diessigsäure-bis-hydrazid (TEDEH).

Gegenstand der vorliegenden Erfindung ist deshalb auch ein Verfahren zur Herstellung der erfindungsgemäßen Cobalamin-Säurehydrazide der Formel (I), das dadurch gekennzeichnet ist, daß man in einer Cobalamin-Carbonsäure der Formel B-COOH die Carboxylgruppe durch Umsetzung mit einem Hydrazid in die Gruppe -CO-NH-NH ( R-CO-NH-NH )ₓ H überführt, wobei, wenn x=1, der Aufbau dieser Gruppierung auch stufenweise erfolgen kann. Nach dem erfindungsgemäßen Verfahren werden insbesondere die nachstehend als bevorzugt angegebenen erfindungsgemäßen Verbindungen der Formel (I) hergestellt.

Die erfindungsgemäßen Carbonsäurehydrazide der Formel (I) sind gut isolierbare und charakterisierbare, beständige Verbindungen, die stabil und deshalb eine gute Lagerfähigkeit besitzen.

Von den erfindungsgemäßen Cobalaminhydraziden der Formel (I) sind diejenigen bevorzugt, in denen der Cobalaminrest B den Rest des Methylcobalamins oder insbesondere des Cyanocobalamins B12 darstellt. Die Säurehydrazidgruppierung -CO-NH-NH- befindet sich vorzugsweise in der b-, e-, und insbesondere in der d-Stellung (entspricht der 3-, 13- und insbesondere der 8-Stellung der -CH₂-CH₂-CO-NH₂-Gruppierung im Corrin-Grundgerüst).

Die Verknüpfungsgruppierung (Spacer) R kann z.B. eine Alkylen-, Aralkylen- oder Arylengruppierung sein, die auch ein oder mehrere Heteroatome enthalten kann. Vorzugsweise bedeutet die Gruppierung -R-CO- einen von einer Alkylendicarbonsäure, in der die Alkylengruppe durch ein oder mehrere Heteroatome, insbesondere NH oder O, unterbrochen sein kann, abgeleiteten Diacylrest. Insbesondere ist die Gruppierunq -R-CO- ein von einer Polyalkylenglykol-, Polyetherpolyol- oder Polyesterpolyol-Dicarbonsäure abgeleiteter Diacylrest, und in erster Linie der von Triethylenglykol-Diessigsäure (TEDE) abgeleitete Diacylrest der Formel 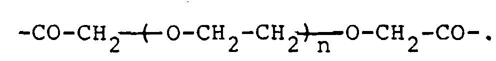 worin n = 3, aber auch der Diacylrest mit n=1 oder 2.

Von den erfindungsgemäßen Verbindungen der Formel (I) sind, insbesondere im Hinblick auf ihre Verwendung zur Herstellung von Cobalamin/Protein-Konjugaten, in erster Linie zu nennen B12-d-CO-NH-NH₂ und B12-d-CO-NH-NH-CO- 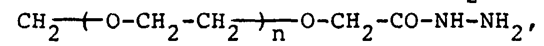 worin n 1 oder 2, und insbesondere 3 bedeutet.

Die erfindungsgemäßen Cobalamin-Säurehydrazide der Formel (I) stellen eine neue Form von aktivierten Cobalaminen dar. So lassen sich die Cobalamine der Formel (I) z.B. direkt und mit hohen Ausbeuten an oxidierte Glykosylreste von Glykoproteinen, wie z.B. Peroxidase (POD) kuppeln. Dabei werden definierte, gut charakterisierbare Konjugate erhalten, die hydrolysestabil sind. Dabei ist gleichzeitig die immunologische Zugänglichkeit sehr hoch. Diese immunologische Zugänglichkeit läßt sich in den erfindungsgemäßen Cobalamin-Konjugaten noch dadurch steigern, daß man die Hydrazofunktion mit einer Spacergruppierung R verlängert (vgl. Formel III, x=1). Auf diese Weise läßt sich z.B. die Bindungsfähigkeit der Cobalamin-Hydrazide (Haptenkomponente) an einen Antikörper noch weiter erhöhen.

Gegenstand der vorliegenden Erfindung sind deshalb auch die Cobalamin-Konjugate der allgemeinen Formel (III) 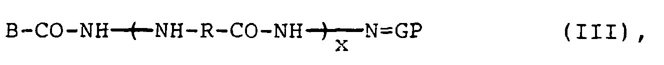 worin B, R und x die in Anspruch 1 angegebene Bedeutung besitzen und GP den Rest eines glykosylgruppenhaltigen Proteins darstellt, das über einen Glykosylrest an die -NH-N=-Gruppierung gebunden ist.

Vorzugsweise ist B der von Cyano-Cobalamin abgeleitete Rest B12, besonders bevorzugt besitzen diese Hydrazide die Formel

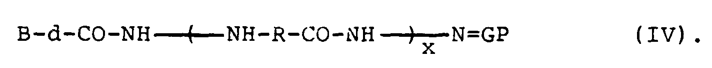

Bei diesen Verbindungen bedeutet -R-CO- den Rest 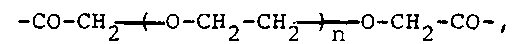 mit n 1, 2 oder 3, insbesondere entsprechen sie der Formel B12-d-CO-NH-N=GP oder der Formel 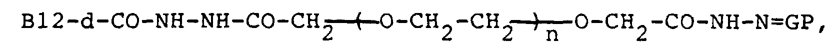 mit n = 1, 2 oder 3.

Der Glykoprotein-Rest GP kann sich von irgendeinem geeigneten Glykoprotein oder Glykoproteid ableiten, wie z.B. von Serumproteinen, Plasmaproteinen, Glykoenzymen, Antikörpern, Mukoproteiden usw. Bevorzugte erfindungsgemäße Cobalamin-Konjugate sind diejenigen, die sich von den vorstehend bevorzugt genannten Cobalamin-Säurehydraziden ableiten. Als Glykoprotein wird vorzugsweise ein Markierungsenzym, wie z.B. Alkalische Phosphatase (AP), und insbesondere Peroxidase (POD) eingesetzt. Im Hinblick auf ihre Eignung zur Verwendung in Immunoassays sind in erster Linie zu nennen: B12-d-CO-NH-N=GP und 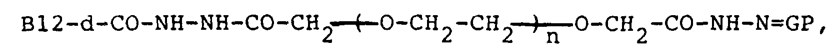 worin n 1 oder 2, und insbesondere 3 ist.

Die Herstellung der erfindungsgemäßen Cobalamin-Konjugate der Formel (III) kann erfolgen durch Kupplung (Kondensation) der Cobalamin-Säurehydrazide der Formel (I) mit den OH-Gruppen von Glykosylresten der Glykoproteine nach deren Oxidation und Ausbildung der Hydrazongruppierung -NH-N=CH-Glykoprotein. Die Reaktionsbedingungen entsprechen dabei den für die Herstellung von Hydrazonen durch Kondensationsreaktion üblichen Bedingungen.

Gegenstand der vorliegenden Erfindung ist deshalb auch ein Verfahren zur Herstellung von Cobalamin-Konjugaten der Formel (III), das dadurch gekennzeichnet ist, daß man ein erfindungsgemäßes Cobalamin-Säurehydrazid der allgemeinen Formel (I) mit einem Glykoprotein in der oxidierten Form unter Bildung eines Hydrazons umsetzt.

Die erfindungsgemäßen Cobalamin-Konjugate der Formel (III) eignen sich hervorragend zur Verwendung bei der immunologischen Bestimmung von Vitamin B12. Unter Verwendung erfindungsgemäßer Cyano-Cobalamin-Konjugate, insbesondere von 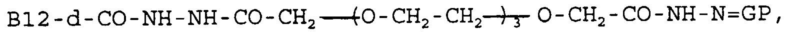 ist z.B. eine reproduzierbare, rasche und einfach durchzuführende Bestimmung für Vitamin B12 (Vitamin B12-Immunoassay) mit hoher Empfindlichkeit und Genauigkeit möglich. Eine derartige Bestimmungsmethode ist Gegenstand der deutschen Patentanmeldung DE 3900650 (Titel: Vitamin-B12-Bestimmung) der gleichen Anmelderin mit dem gleichen Anmeldetag, deren Inhalt Gegenstand der vorliegenden Anmeldung ist.

Es ist jedoch auch möglich, die erfindungsgemäßen Cobalamin-Konjugate in bekannten Verfahren zur Bestimmung von Vitamin B12 anstelle eines radioaktiv markierten Vitamin B12 einzusetzen. Derartige geeignete Radioimmunoassays sind beispielsweise beschrieben in Clinical Biochemiscry 18 (1985) 261-266, USP 3,981,863, Clinica Chimica Acta 56 (1974) 143-149, Lit.Clin.Path. 20 (1967) 683-686, Brit.J.Hämat. 22 (1972), 21-31.

Die nachfolgenden Beispiele und die Abbildung erläutern die Erfindung weiter.

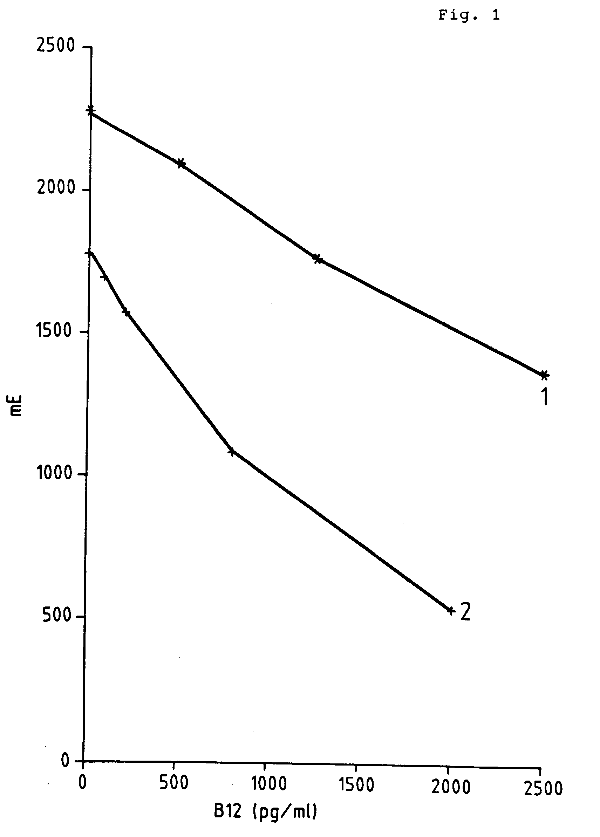

- Fig. 1: zeigt einen Vergleich der Eichkurven für einen Enzymimmunoassay für Vitamin B12 unter Verwendung eines erfindungsgemäßen B12-Enzymkonjugats nach Beispiel 5 (Kurve 2, Aktivität 20 mU/ml) und eines Tests unter Verwendung eines Konjugats nach Beispiel 6 (Kurve 1, Aktivität 90 mU/ml). Meßwellenlänge: 425 nm, E: Extinktion.

### Beispiele

### Beispiel 1:

### Herstellung Triethylenglykol-diessigsäure-bis-hydrazid (TEDEH)

Das TEDEH wurde nach dem folgenden Reaktionsschema hergestellt:

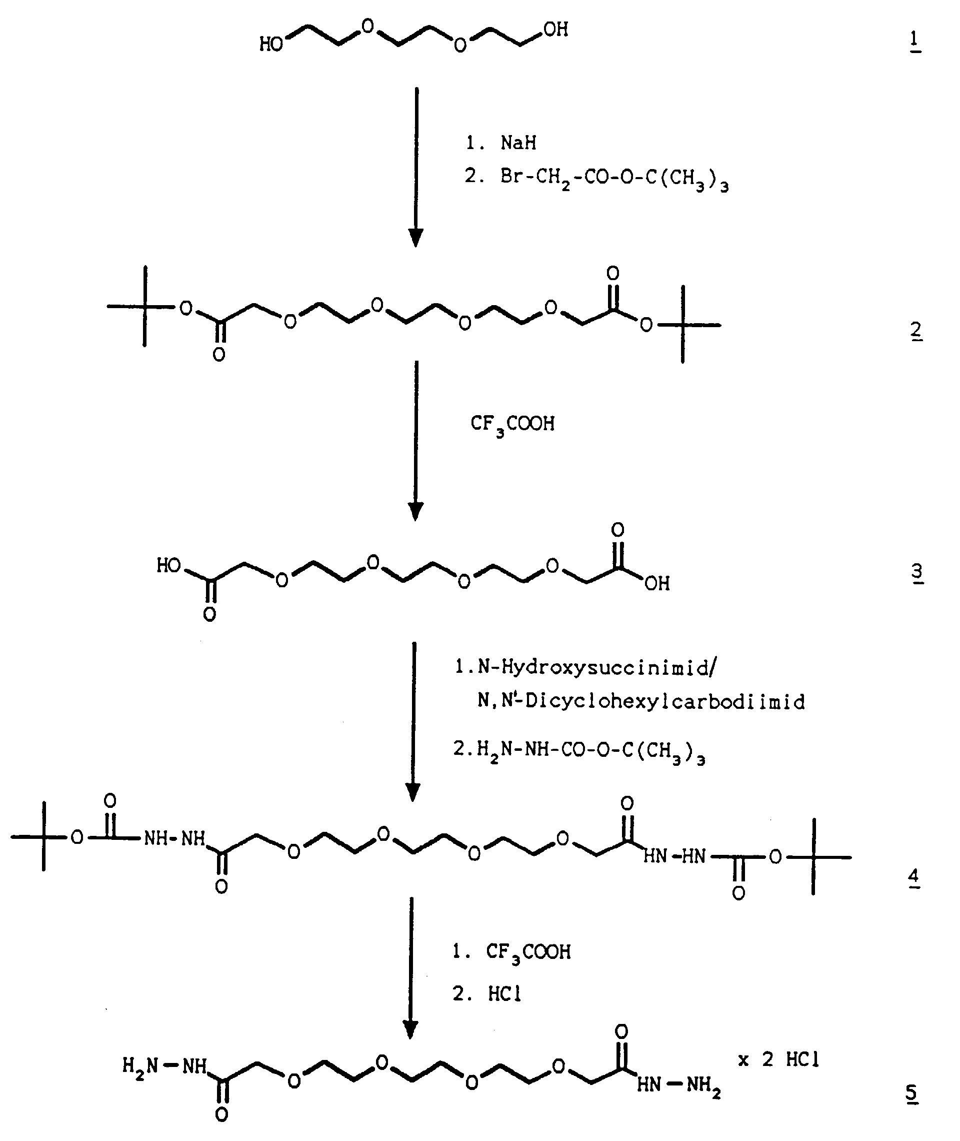

### a) Triethylenglykol-diessigsäure-bis-tert.-butylester (2)

33 g (0,22 mol) wasserfreies Triethylenglykol (1) werden in 300 ml absolutem Dioxan gelöst und unter Rühren bei Raumtemperatur portionsweise mit 12 g (0,4 mol) Natriumhydrid (20 % Paraffinöl enthaltend) versetzt. Dabei tritt leichte Erwärmung auf ca. 45°C ein. Die Suspension wird 3 Stunden bei Raumtemperatur gerührt. Danach läßt man unter Eiskühlung innerhalb von einer Stunde 78 g (0,4 mol) Bromessigsäure-tert.-butylester zutropfen. Die Suspension wird weitere 16 Stunden bei Raumtemperatur gerührt. Anschließend wird das ausgefallene NaBr über eine Filternutsche abgetrennt und das Filtrat am Wasserstrahlvakuum eingedampft. Den öligen Rückstand löst man in 0,5 l Essigester, wäscht mit 0,5 l Wasser und trocknet die organische Phase mit 30 g Na₂SO₄. Die Lösung wird bis auf ein Restvolumen von etwa 100 ml eingedampft und auf eine Kieselgelsäule (8,5 x 55 cm) aufgetragen. Man eluiert mit Essigester und prüft die einzelnen Fraktionen durch analytische Dünnschichtchromatographie (Kieselgel, Laufmittel: Essigester). Die Polyethylenglykolderivat-haltigen Fraktionen werden durch Besprühen mit Dragendorff-Reagenz (K. Thoma et al., Sci. Pharm. 32 (1964), 216) sichtbar gemacht. Die das Produkt (2) (R_{f} = 0,60) enthaltenden Fraktionen werden vereinigt und am Wasserstrahlvakuum eingedampft.
- Ausbeute:: 19,2 g farbloses Öl (23 % d.Th.).

### b) Triethylenglykol-diessigsäure (3)

18,9 g (50 mmol) Ester (2) werden bei Raumtemperatur in 30 ml Trifluoressigsäure gelöst und zwei Stunden gerührt. Die Lösung dampft man im Wasserstrahlvakuum bei 35°C ein, digeriert den Rückstand dreimal mit je 100 ml Diethylether und trocknet im Hochvakuum bei 45°C. Das Produkt (3) kann ohne weitere Reinigung weiterverwendet werden.
- Ausbeute:: 11,2 g farbloses Öl (84 % d.Th.).
- DC:: Kieselgel, Laufmittel: Essigester/Methanol- 4/1; R_{f} = 0,08.

### c) Triethylenglykol-diessigsäure-bis-(BOC-hydrazid) (4)

8,0 g (30 mmol) der Dicarbonsäure (3) werden zusammen mit 8,28 g (72 mmol) N-Hydroxysuccinimid in 200 ml trockenem Tetrahydrofuran gelöst und mit 14,8 g (72 mmol) N,N′-Dicyclohexylcarbodiimid in 50 ml trockenem Tetrahydrofuran unter Wasserbad-Kühlung bei 20°C versetzt. Die Lösung läßt man 20 Stunden bei Raumtemperatur rühren, danach wird vom ausgefallenen Dicyclohexylharnstoff abfiltriert und im Vakuum eingedampft. Der zähflüssige Rückstand wird in 100 ml Essigester gelöst, die Lösung filtriert und abermals eingedampft. Der letzte Vorgang wird noch zweimal wiederholt, dann wird der Rückstand mit 50 ml Isopropanol digeriert und anschließend im Hochvakuum getrocknet. Das erhaltene zähflüssige Öl (ca. 9 g) wird in 60 ml wasserfreiem Chloroform gelöst und unter Rühren bei Raumtemperatur mit 7,9 g (60 mmol) tert.-Butylcarbazat (BOC-Hydrazin) versetzt. Die Reaktionsmischung, die sich anfangs leicht erwärmt, läßt man 20 Stunden bei Raumtemperatur rühren. Danach wäscht man zweimal mit je 100 ml Wasser, trocknet mit 5 g Na₂SO₄ und dampft die Lösung im Wasserstrahlvakuum ein.
- Ausbeute:: 9,2 g zähflüssiges, leicht gelbliches Öl (60 % d.Th.).
- DC:: Kieselgel, Laufmittel: Essigester/Methanol=4/1; R_{f} = 0,52.

### d) Triethylenglykol-diessigsäure-bis-hydrazid-Hydrochlorid (5)

5,1 g (10 mmol) der Verbindung (4) werden in 20 ml Trifluoressigsäure gelöst und 2 Stunden bei Raumtemperatur gerührt. Danach wird die Lösung im Wasserstrahlvakuum bei 35°C eingedampft, der Rückstand in 80 ml wasserfreiem Essigester gelöst und unter Kühlung bei 0°C 10 Minuten HCl-Gas durch die Lösung geleitet. Hierbei bildet sich ein Niederschlag von festem Carbonsäurehydrazid-Hydrochlorid (5). Die Suspension läßt man 2 Stunden bei 0°C stehen, saugt anschließend ab und wäscht das erhaltene, hygroskopische Salz mit 40 ml wasserfreiem Essigester. Es wird im Exsikkator unter Hochvakuum über CaCl₂ getrocknet.
- Ausbeute:: 2,8 g leicht bräunliches Pulver (75% d.Th.).
- DC:: Kieselgel, n-Butanol/Eisessig/Wasser=50/15/25; R_{f}=0,45.

### Beispiel 2:

### Cyanocobalamin-d-Säurehydrazid (B12-d-CO-NH-NH₂)

135 mg (0,1 mmol) Cyanocobalamin-d-säure werden zusammen mit 46 mg (0,4 mmol) N-Hydroxysuccinimid in 10 ml eines Gemisches von Dimethylformamid/Wasser=1:1 gelöst und mit 98 mg NaCN versetzt. Die Lösung wird mit 1 mol/l NaOH auf pH 6 gestellt. Zu der Lösung gibt man 77 mg (0,4 mmol) N-Ethyl-N′-dimethylaminopropyl-carbodiimid-Hydrochlorid (EDC) und 650 mg (5 mmol) Hydraziniumsulfat. Der pH-Wert wird mit NaOH nachgestellt, anschliessend dunkelt man das Reaktionsgefäß ab und läßt die Lösung bei Raumtemperatur rühren. In Abständen von 6 bis 14 Stunden gibt man insgesamt fünfmal je 46 mg N-Hydroxysuccinimid und 77 mg EDC zur Reaktionslösung, dabei wird jedesmal der pH-Wert auf 6 nachgestellt. Nach einer Gesamtreaktionszeit von 4 Tagen wird am Hochvakuum eingedampft, der Rückstand mit 10 ml Tetrahydrofuran digeriert und über eine Filternutsche abgesaugt. Der feste Rückstand wird in 10 ml Wasser gelöst und auf eine Chromatographiesäule (Material: Styroldivinylbenzolcopolymer) Amberlite®-XAD-2 (V=100 ml) aufgetragen. Die salzförmigen Verunreinigungen werden mit 300 ml Wasser von der Säule gewaschen und anschließend das Rohprodukt mit 300 ml Methanol eluiert. Die Lösung dampft man am Wasserstrahlvakuum ein, löst den Rückstand in 10 ml Wasser und gibt sie auf eine Dowex®-1x2-Säule (saurer Ionenaustauscher, Acetat-Form, 80 ml). Das Endprodukt wird mit 250 ml Wasser eluiert, dabei bleibt nicht umgesetzte Säure auf der Säule zurück und kann im Anschluß mit 0,04 mol/l Natriumacetat-Puffer (pH 4,7) eluiert werden. Die das Produkt enthaltenden Fraktionen werden vereinigt und lyophilisiert.
- Ausbeute:: 84 mg kirschrotes Pulver (60% d.Th.).

Auf die gleiche Weise erhält man, ausgehend von Methylcobalamin-d-säure, das Methylcobalamin-d-Säure-hydrazid.

### Beispiel 3:

### Herstellung von Cyanocobalamin-d-Säurehydrazid-POD-Konjugat (B12-d-CO-NH-N=POD)

30 mg Meerrettich-Peroxidase (POD, EC 1.11.1.7) werden in 4,5 ml 0,03 mol/l Natriumacetat-Puffer pH 5,5 gelöst. Man gibt 0,6 ml 0,2 mol/l Natriumperiodat-Lösung (in 0,03 mol/l Natriumacetat-Puffer pH 5,5) zu und läßt eine Stunde bei Raumtemperatur rühren. Danach wird die Lösung auf eine Sephadex-G-25-Säule (Molekularsieb, V=50 ml) aufgetragen und mit 0,03 mol/l Natriumacetat-Puffer (pH 5,5) eluiert (Detektion bei 403 nm). Die proteinhaltigen Fraktionen werden vereinigt und mit 2,7 mg B12-d-CO-NH-NH₂ aus Beispiel 2 versetzt. Man läßt 16 Stunden bei Raumtemperatur rühren und zieht das Gemisch auf eine Ultrogel-AcA-202-Säule (Molekularsieb, V=80 ml) auf. Es wird mit 0,03 mol/l Natriumacetat-Puffer pH 5,5 eluiert (Detektion ν = 403 nm). Die das Endprodukt enthaltenden Fraktionen werden vereinigt, 6 Stunden gegen Wasser dialysiert und anschließend lyophilisiert.

Ausbeute: 26 mg (87 % d.Th.).

### Beispiel 4:

Herstellung von Cyancobalamin-d-Säure-TEDEH

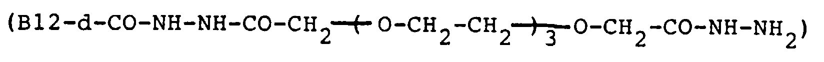

135 mg (0,1 mmol) Cyancobalamin-d-säure werden zusammen mit 46 mg (0,4 mmol) N-Hydroxysuccinimid in 10 ml eines Gemisches von Dimethylformamid-Wasser 1:1 gelöst und mit 98 mg NaCN versetzt. Die Lösung wird mit 1 mol/l NaOH auf pH 5,5 gestellt. Zu der Lösung gibt man 77 mg (0,4 mmol) N-Ethyl-N′-dimethylaminopropyl-carbodiimid-Hydrochlorid (EDC) und 1,84 g (5 mmol) TEDEH-Hydrochlorid. Der pH-Wert wird mit NaOH nachgestellt, anschließend dunkelt man das Reaktionsgefäß ab und läßt die Lösung bei Raumtemperatur rühren. In Abständen von 6 bis 14 Stunden gibt man insgesamt fünfmal je 46 mg N-Hydroxysuccinimid und 77 mg EDC zur Reaktionslösung, dabei wird jedesmal der pH-Wert auf 5,5 nachgestellt. Nach einer Gesamtreaktionszeit von 4 Tagen wird im Hochvakuum eingedampft, der Rückstand mit 10 ml Aceton digeriert und das Lösungsmittel dekantiert. Der nahezu feste Rückstand wird in 10 ml Wasser gelöst und auf eine Amberlite®-XAD-2-Säule (V=100 ml) aufgetragen. Die salzförmigen Verunreinigungen werden mit 300 ml Wasser von der Säule gewaschen und anschließend das Rohprodukt mit 300 ml Methanol eluiert. Die Lösung dampft man am Wasserstrahlvakuum ein, löst den Rückstand in 10 ml Wasser und gibt auf eine Dowex®-1x2-Säule (Acetat-Form, 80 ml). Das Endprodukt wird mit 250 ml Wasser eluiert, dabei bleibt nicht umgesetzte Säure auf der Säule zurück und kann im Anschluß mit 0,04 mol/l Natriumacetat-Puffer, pH 4,7 eluiert werden. Die das Produkt enthaltenden Fraktionen werden vereinigt und lyophilisiert.
- Ausbeute:: 75 mg kirschrotes, leicht hygroskopisches Pulver (46 % d.Th.).

Auf die gleiche Weise erhält man, ausgehend von Methylcobalamin-d-säure, das Methylcobalamin-d-Säure-TEDEH.

### Beispiel 5:

Herstellung von Cyancobalamin-d-Säure-TEDEH-POD-Konjugat

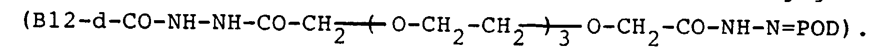

30 mg Meerrettich-Peroxidase werden in 4,5 ml 0,03 mol/l Natriumacetat-Puffer pH 5,5 gelöst. Man gibt 0,6 ml 0,2 mol/l Natriumperjodat-Lösung (in 0,03 mol/l Natriumacetat-Puffer pH 5,5) zu und läßt eine Stunde bei Raumtemperatur rühren. Danach wird die Lösung auf eine Sephadex®-G-25-Säule (V=50 ml) aufgetragen und mit 0,03 mol/l Natriumacetat-Puffer (pH 5,5) eluiert (Detektion bei ν= 403 nm). Die proteinhaltigen Fraktionen werden vereinigt und mit 2,7 mg des Hydrazids aus Beispiel 4 versetzt. Man läßt 16 Stunden bei Raumtemperatur rühren und zieht das Gemisch auf eine Ultrogel®-AcA-202-Säule (V=80 ml) auf. Es wird mit 0,03 mol/l Natriumacetat-Puffer pH 5,5 eluiert (Detektion bei ν= 403 nm). Die das Endprodukt enthaltenden Fraktionen werden vereinigt, 6 Stunden gegen Wasser dialysiert und anschließend lyophilisiert.

Ausbeute: 27 mg (90% d.Th.).

Analog zu den in den vorstehenden Beispielen 3 und 5 beschriebenen Verfahren zur Herstellung von POD-Konjugaten lassen sich unter Verwendung anderer glykosylierter Markerenzyme, wie z.B. von alkalischer Phosphatase (AP) die entsprechenden Konjugate herstellen.

### Beispiel 6

### Herstellung von Cyanocobalamin-d-Monocarbonsäure-POD-Konjugat (Vergleichsverbindung)

Die Herstellung erfolgt wie in Clin.Chim.Acta 56 (1974), 143-149 beschrieben.

Dazu wird B12-Monocarbonsäure, wie in J.Am.Chem.Soc. 102 (1980) 2215 beschrieben, hergestellt.

100 mg POD und 10 mg Vitamin B12 werden in 2,5 ml Phosphatpuffer (0,07 mol/l, pH 8,2) gelöst und 25 mg Carbodiimid zugegeben. Die Lösung wird für 72 Stunden bei 4°C gerührt und anschließend gegen deionisiertes Wasser für 72 Stunden dialysiert.

Die Aufreinigung des Konjugats erfolgt wie in Beipiel 5 beschrieben.

### Beispiel 7

### Bestimmung von Vitamin B12

### a) Probenvorbereitung

250 µl Humanserum werden mit 125 µl Ablösereagenz (bestehend aus 8 mg/ml Liponsäure, 1 mg/ml Kaliumcyanid, gelöst in 0,5 mol/l NaOH) gemischt und 15 min bei Raumtemperatur inkubiert. Anschließend werden 125 µl, 200 mmol/l Phosphatpuffer, pH 4,1 zugegeben.

### b) Reagenzien

Polystyrolröhrchen beschichtet mit Thermo-RSA Streptavidin (hergestellt nach EP-A 0269092)

### Reagenz 1

95 ng/ml biotinylierter monoklonaler Antikörper gegen Vitamin B12 (Biotinylierung nach JACS 100 (1978) 3585-3590).

Zellinien, die geeignete monoklonale Antikörper produzieren, sind bei der European Collection of Animal Cell Culture (ECACC) Porton Down, GB, unter den Nummern ECACC 88101301 und ECACC 88101302 hinterlegt.

40 mmol/l Phosphatpuffer, pH 7,2.

### Reagenz 2

B12-d-CO-NH-NH-CO-CH2-(-O-CH2-CH2-)₃-O-CH2-CO-NH-N = POD) (Aktivität ca. 20 mU/ml)

40 mmol/l Phosphatpuffer, pH 7,2.

### Reagenz 3

100 mmol/l Phosphat-Citratpuffer, pH 4,4.
1,9 mmol/l ABTS^{R}
(2,2˝-Azino-di[3-ethyl-benzthiazolin-sulfonat])
3,2 mmol/l Natriumperborat

### c) Durchführung der Bestimmung

Zur Durchführung der Bestimmung werden 200 µl Probe mit 800 µl Reagenz 1 in ein Streptavidin-tube gegeben und 60 min bei Raumtemperatur inkubiert. Anschließend wird mit Waschlösung (250 mg/ml Natriumchlorid, 1 mg/100 ml Kupfersulfat) gewaschen und 1000 µl Reagenz 2 zugegeben und 30 min bei Raumtemperatur inkubiert. Es wird mit Waschlösung (250 mg/ml Natriumchlorid, 1 mg/100 ml Kupfersulfat) gewaschen und 1000 µl Reagenz 3 zugegeben, für 30 min bei Raumtemperatur inkubiert und die gebildete Farbe als Maß für den Vitamin B12-Gehalt bei 420 nm gemessen.

Fig. 1 zeigt die dabei erhaltene Eichkurve im Vergleich zu einer Eichkurve für einen Test nach Beispiel 6. Zur Ermittlung der Eichkurve werden anstelle der Serumprobe Standards verwendet, die aus Cyanocobalamin in 40 mmol/l Phosphatpuffer, pH 7,2 mit 0,9 % Natriumchlorid, 0,9 % Crotein C und 0,1 % Kaliumchlorid bestehen.

### Beispiel 8

### Bestimmung von Vitamin B12 (Vergleichsbeispiel)

Es wird eine Vitamin B12-Bestimmung wie in Beispiel 7 beschrieben durchgeführt, wobei die dort genannten Standards verwendet werden. Anstelle des in Reagenz 2 verwendeten erfindungsgemäßen Konjugats, wird ein POD-Konjugat nach Beispiel 6 verwendet mit einer Aktivität von ca. 90 mU/ml.

Figur 1 zeigt den Vergleich der Eichkurven einer Vitamin B12-Bestimmung nach Beispiel 7 und Beispiel 8. Danach zeigt sich, daß mit dem Konjugat nach Beispiel 6 trotz höherer Aktivität eine flachere Eichkurve erhalten wird als mit dem erfindungsgemäßen Konjugat.

## Patentansprüche (Patentansprüche für folgende(n) Vertragsstaat(en): AT, BE, CH, LI, DE, DK, FR, GB, GR, IT, LU, NL, SE)

1. Cobalamin-Säurehydrazide der Formel 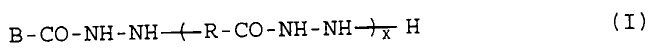 worin B den aus Cobalaminen der Formel 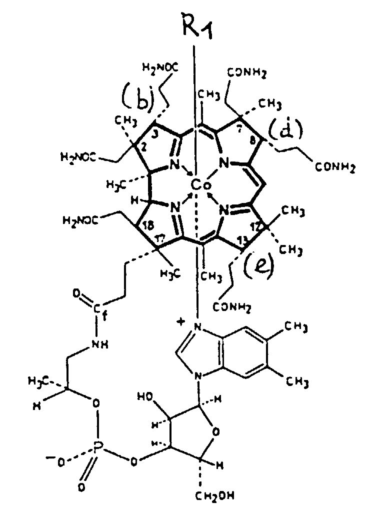 durch Abspaltung einer -CONH₂-Gruppe gebildeten Rest bedeutet, wobei R₁ die verschiedenen, die einzelnen Cobalamine unterscheidenden Reste darstellt, R eine Verknüpfungsgruppierung (Spacer), ausgewählt aus Alkylen, Aralkylen oder Arylen, die ein oder mehrere Heteroatome enthalten kann, bedeutet, und x 0 oder 1 ist.

2. Cobalamin-Säurehydrazide nach Anspruch 1,
**dadurch gekennzeichnet,**
daß R -CO-CH₂-(O-CH₂-CH₂-)ₙO-CH₂ ist, wobei n 1, 2 oder 3 ist.

3. Verfahren zur Herstellung von Cobalamin-Säurehydraziden der Formel (I) nach Anspruch 1 oder 2,
**dadurch gekennzeichnet,**
daß man in einer Cobalamin-Carbonsäure der Formel B-COOH die Carboxylgruppe durch Umsetzung mit einem Hydrazid in die Gruppe 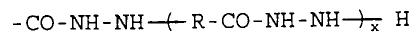 überführt, wobei, wenn x = 1 ist, der Aufbau dieser Gruppierung auch stufenweise erfolgen kann.

4. Cobalamin-Konjugate der Formel (III) 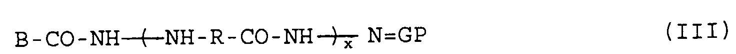 worin B, R und x die in Anspruch 1 oder 2 angegebene Bedeutung besitzen und GP den Rest eines glykosylgruppenhaltigen Proteins darstellt, das über einen Glykosylrest an die -NH-N=-Gruppierung gebunden ist.

5. Cobalamin-Konjugate nach Anspruch 4,
**dadurch gekennzeichnet,**
daß GP den Rest eines Markierungsenzyms darstellt.

6. Verfahren zur Herstellung von Cobalamin-Konjugaten gemäß Anspruch 4 oder 5,
**dadurch gekennzeichnet,**
daß man ein Cobalamin-Säurehydrazid der allgemeinen Formel (I) gemäß Anspruch 1 oder 2 mit einem Glykoprotein in der oxidierten Form unter Bildung eines Hydrazons umsetzt.

7. Verwendung eines Cobalamin-Säurehydrazids nach Anspruch 1 oder 2 zur Herstellung von Cobalamin-Konjugaten, insbesondere von Cobalamin/Glykoprotein-Konjugaten gemäß Anspruch 4.

8. Verwendung eines Cobalamin-Konjugates nach Anspruch 4 oder 5 für immunologische Untersuchungs- und Analysenmethoden, insbesondere in Immunoassays für die Bestimmung von Cyano-cobalamin.

9. Triethylenglykol-diessigsäure-bis-hydrazid (TEDEH).

10. Verfahren zur Herstellung von Triethylenglykol-diessigsäuresäure-bis-hydrazid (TEDEH),
**dadurch gekennzeichnet,**
daß man Triethylenglykol durch Umsetzung mit Bromessigsäure- tert.-butylester in den Triethylenglykol-diessigsäure-bis-tert.-butylester überführt, diesen dann in die freie Dicarbonsäure überführt, die freie Dicarbonsäure mit tert.-Butyloxycarbonylhydrazin in das Triethylenglykoldiessigsäure-bis-(BOC-hydrazid) überführt, und das so erhaltene Reaktionsprodukt durch Abspaltung der BOC-Reste in Triethylenglykoldiessigsäure-bis-hydrazid bzw. das entsprechende Hydrochlorid überführt.

11. Verwendung von Triethylenglykol-diessigsäure-bis-hydrazid (TEDEH) zur Einführung einer Spacer-Gruppierung in CobalaminKonjugate der Formel (III) gemäß Anspruch 4.

## Patentansprüche (Patentansprüche für folgende(n) Vertragsstaat(en): ES)

1. Verfahren zur Herstellung von Cobalamin-Säurehydraziden der Formel (I) 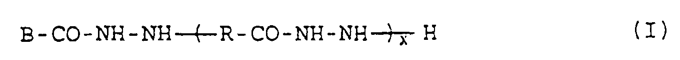 worin B den aus Cobalaminen der Formel 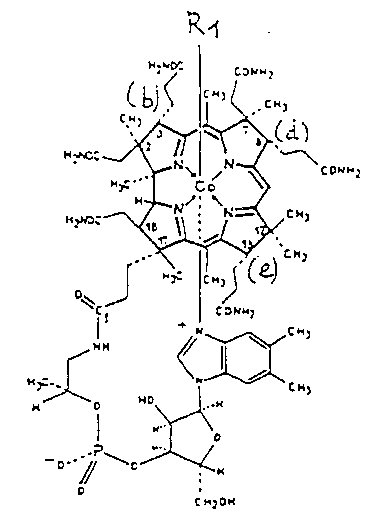 durch Abspaltung einer -CONH₂-Gruppe gebildeten Rest bedeutet, wobei R₁ die verschiedenen, die einzelnen Cobalamine unterscheidenden Reste darstellt, R eine Verknüpfungsgruppierung (Spacer), ausgewählt aus Alkylen, Aralkylen oder Arylen, die ein oder mehrere Heteroatome enthalten kann, bedeutet, und x 0 oder 1 ist.

2. Verfahren nach Anspruch 1,
dadurch gekennzeichnet,
daß R-CO-CH₂-(O-CH₂-CH₂-)ₙO-CH₂ ist, wobei n 1, 2 oder 3 ist.

3. Verfahren zur Herstellung von Cobalamin-Konjugaten der Formel (III) 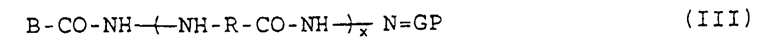 worin B, R und x die in Anspruch 1 oder 2 angegebene Bedeutung besitzen und GP den Rest eines glykosylgruppenhaltigen Proteins darstellt, das über einen Glykosylrest an die -NH-N=-Gruppierung gebunden ist.

4. Verfahren nach Anspruch 3,
dadurch gekennzeichnet,
daß GP den Rest eines Markierungsenzyms darstellt.

5. Verwendung eines Cobalamin-Säurehydrazids nach Anspruch 1 oder 2 zur Herstellung von Cobalamin-Konjugaten, insbesondere von Cobalamin/Glykoprotein-Konjugaten gemäß Anspruch 3.

6. Verwendung eines Cobalamin-Konjugates nach Anspruch 3 oder 4 für immunologische Untersuchungs- und Analysenmethoden, insbesondere in Immunoassays für die Bestimmung von Cyano-cobalamin.

7. Verfahren zur Herstellung von Triethylenglykol-diessigsäuresäure-bis-hydrazid (TEDEH),
dadurch gekennzeichnet,
daß man Triethylenglykol durch Umsetzung mit Bromessigsäure- tert.-butylester in den Triethylenglykol-diessigsäure-bis-tert.-butylescer überführt, diesen dann in die freie Dicarbonsäure überführt, die freie Dicarbonsäure mit tert.-Butyloxycarbonylhydrazin in das Triethylenglykoldiessigsäure-bis-(BOC-hydrazid) überführt, und das so erhaltene Reaktionsprodukt durch Abspaltung der BOC-Reste in Triethylenglykoldiessigsäure-bis-hydrazid bzw. das entsprechende Hydrochlorid überführt.

8. Verwendung von Triethylenglykol-diessigsäure-bis-hydrazid (TEDEH) zur Einführung einer Spacer-Gruppierung in Cobalamin-Konjugate der Formel (III) gemäß Anspruch 3.

## Claims (Claims for the following Contracting State(s): AT, BE, CH, LI, DE, DK, FR, GB, GR, IT, LU, NL, SE)

1. Cobalamin acid hydrazides of the formula 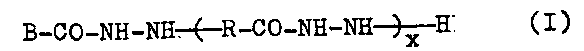 wherein B signifies the residue formed from cobalamins of the formula 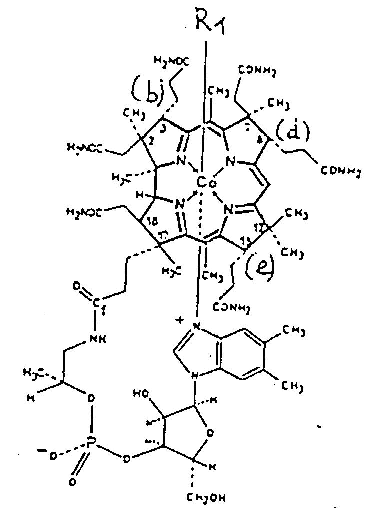 by splitting off of a -CONH₂ group, whereby R₁ represents the various radicals differentiating the individual cobalamins, R signifies a connecting grouping (spacer) selected from alkylene, aralkylene or arylene, which can contain one or more heteroatoms and x is O or 1,

2. Cobalamin acid hydrazides according to claim 1, characterised in that R is 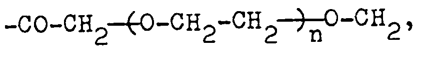 whereby n is 1, 2 or 3.

3. Process for the preparation of cobalamin acid hydrazides of the formula (I) according to claim 1 or 2, characterised in that, in a cobalamin carboxylic acid of the formula B-COOH, one converts the carboxyl group by reaction with a hydrazide into the group 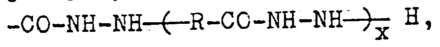 whereby, when x = 1, the build up of this grouping can also take place stepwise.

4. Cobalamin conjugates of the formula (II) 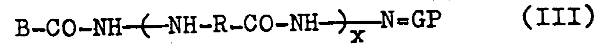 wherein B, R and x possess the meanings given in claim 1 or 2 and GP represents the residue of a glycosyl group-containing protein which is bound via a glycosyl residue to the -NH-N= grouping.

5. Cobalamin conjugates according to claim 4, characterised in that GP represents the residue of a labelling enzyme.

6. Process for the preparation of cobalamin conjugates according to claim 4 or 5, characterised in that one reacts a cobalamin acid hydrazide of the general formula (I) according to claim 1 or 2 with a glycoprotein in the oxidised form with formation of a hydrazone..

7. Use of a cobalamin acid hydrazide according to claim 1 or 2 for the preparation cobalamin conjugates, especially of cobalamin/glycoprotein conjugates according to claim 4.

8. Use of a cobalamin conjugate according to claim 4 or 5 for immunological investigation and analysis methods, especially in immunoassays for the determination of cyanocobalamain.

9. Triethyleneglycol-diacetic acid bis-hydrazide (TEDEH).

10. Process for the preparation of triethyleneglycoldiacetic acid bis-hydrazide (TEDEH), characterised in that one converts triethyleneglycol by reaction with bromoacetic acid tert.-butyl ester into triethyleneglycol-diacetic acid bis-tert.-butyl ester, then converts this into the free dicarboxylic acid, converts the free dicarboxylic acid with tert.-butyloxy carbonylhydrazine into triethyleneglycol-diacetic acid bis-(HOC-hydrazide) and converts the so-obtained 5 reaction product, by splitting off of the BOC residue, into triethyleneglycol-diacetic acid bis-hydrazide or the corresponding hydrochloride.

11. Use of triethyleneglycol-diacetic acid bis-hydrazide (TEDEH) for the introduction of a spacer grouping into cobalamin conjugates of the formula (III) according to claim 4.

## Claims (Claims for the following Contracting State(s): ES)

1. Process for the preparation of cobalamin acid hydrazides of the formula I 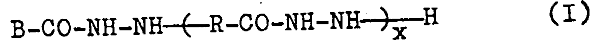 wherein B signifies the residue formed from cobalamins of the formula (I) 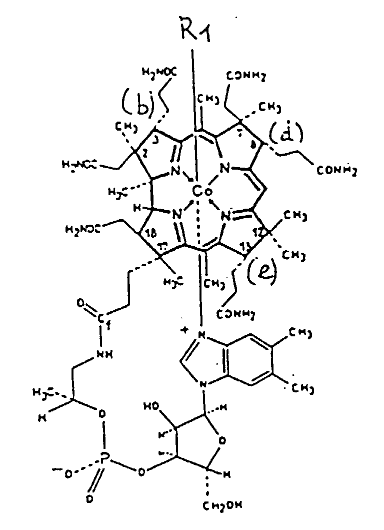 by splitting off a -CONH₂ group, whereby R₁ represents the various radicals differentiating the individual cobalamins, R signifies a connecting grouping (spacer) selected from alkylene, aralkylene or arylene, which can contain one or more heteroatoms, and x is O or 1.

2. Process according to claim 1, characterised in that R is 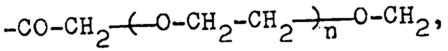 whereby n is 1, 2 or 3.

3. Process for the preparation of cobalamin conjugates of the formula (III) 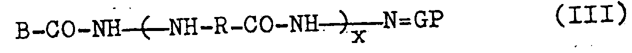 wherein B, R and x possess the meaning given in claim 1 or 2 and GP represents the residue of a glycosyl group-containing protein which is bound via a glycosyl residue to the -NH-N= grouping.

4. Process according to claim 3, characterised in that GP represents the residue of a labelling enzyme.

5. Use of a cobalamin acid hydrazide according to claim 1 or 2 for the preparation of cobalamin conjugates, especially of cobalamin/glycoprotein conjugates according to claim 3.

6. Use of a cobalamin conjugate according to claim 3 or 4 for immunological investigation and analysis methods, especially in immunoassays for the determination of cyanocobalamin.

7. Process four the preparation of triethyleneglycoldiacetic acid bis-hydrazide (TEDEH), characterised in that one converts triethyleneglycol by reaction with bromoacetic acid tert.-butyl ester into triethyleneglycol-diacetic acid bis-tert.-butyl ester, then converts this into the free dicarboxylic acid. converts the free dicarboxylic acid with tert.-butyloxycarbonylhydrazine into triethyleneglycoldiacetic acid bis-(BOC-hydrazide) and converts the so obtained reaction product, by splitting off of the BOC residue, into triethyleneglycol-discetic acid bis-hydrazide or the corresponding hydrochloride.

8. Use of triethyleneglycol-diacetic acid bis-hydrazide (TEDEH) for the introduction of a spacer grouping into cobalamin conjugates of the formula (III) according to claim 3.

## Revendications (Revendications pour l'(les) Etat(s) contractant(s) suivant(s): AT, BE, CH, LI, DE, DK, FR, GB, GR, IT, LU, NL, SE)

1. Hydrazides d'acides de cobalamine de formule
B-CO-NH-NH-(-R-CO-NH-NH-)ₓ-H (I)
dans laquelle B représente le reste formé à partir de cobalamines de formule 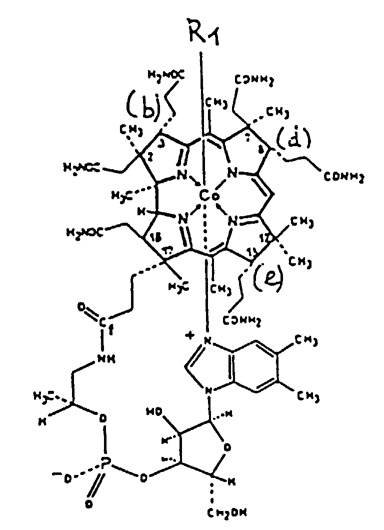 par clivage d'un groupe -CONH₂, R₁ représentant les différents restes qui différencient les diverses cobalamines, R représente un groupement de liaison (espaceur), choisi parmi alkylène, aralkylène et arylène, qui peut contenir un ou plusieurs hétéroatomes, et x est 0 ou 1.

2. Hydrazides d'acides de cobalamine selon la revendication 1, caractérisés en ce que R est -CO-CH₂-(O-CH₂-CH₂-)ₙO-CH₂ où n est 1, 2 ou 3.

3. Procédé de préparation d'hydrazides d'acides de cobalamine de formule (I) selon la revendication 1 ou 2, caractérisé en ce que, dans un acide carboxylique de cobalamine de formule B-COOH, on convertit le groupe carboxyle en le groupe -CO-NH-NH-(-R-CO-NH-NH-)ₓ-H par réaction avec un hydrazide, la construction de ce groupement pouvant aussi avoir lieu par étapes lorsque x = 1.

4. Conjugués de cobalamine de formule (III)
B-CO-NH-(-NH-R-CO-NH-)ₓ-N=GP (III)
où B, R et x possèdent la signification indiquée dans la revendication 1 ou 2 et GP représente le reste d'une protéine contenant des groupes glycosyle qui est liée par le biais d'un reste glycosyle au groupement -NH-N=.

5. Conjugués de cobalamine selon la revendication 4, caractérisés en ce que GP représente le reste d'une enzyme de marquage.

6. Procédé de préparation de conjugués de cobalamine selon la revendication 4 ou 5, caractérisé en ce que l'on fait réagir un hydrazide d'acide de cobalamine de formule générale (I) selon la revendication 1 ou 2 avec une glycoprotéine sous la forme oxydée avec formation d'une hydrazone.

7. Utilisation d'un hydrazide d'acide de cobalamine selon la revendication 1 ou 2 pour la préparation de conjugués de cobalamine, en particulier de conjugués cobalamine/glycoprotéine selon la revendication 4.

8. Utilisation d'un conjugué de cobalamine selon la revendication 4 ou 5 pour des méthodes immunologiques d'examen et d'analyse, en particulier dans des immunodosages pour la détermination de la cyanocobalamine.

9. Bis-hydrazide d'acide triéthyléneglycoldiacétique (TEDEH).

10. Procédé de préparation de bis-hydrazide d'acide triéthylèneglycoldiacétique (TEDEH), caractérisé en ce que l'on convertit le triéthylèneglycol par réaction avec le bromoacétate de tert.butyle en le triéthylèneglycoldiacétate de bis-tert.butyle, puis on convertit celui-ci en l'acide dicarboxylique libre, on convertit l'acide dicarboxylique libre avec la tert.butyloxycarbonylhydrazine en le bis-(BOC-hydrazide) d'acide triéthyléneglycoldiacétique, et on convertit le produit réactionnel ainsi obtenu en le bis-hydrazide d'acide triéthyléneglycoldiacétique ou en le chlorhydrate correspondant par clivage des restes BOC.

11. Utilisation du bis-hydrazide d'acide triéthyléneglycoldiacétique (TEDEH) pour introduire un groupement espaceur dans des conjugués de cobalamine de formule (III) selon la revendication 4.

## Revendications (Revendications pour l'(les) Etat(s) contractant(s) suivant(s): ES)

1. Procédé de préparation d'hydrazides d'acides de cobalamine de formule
B-CO-NH-NH-(-R-CO-NH-NH-)ₓ-H (I)
dans laquelle B représente le reste formé à partir de cobalamines de formule 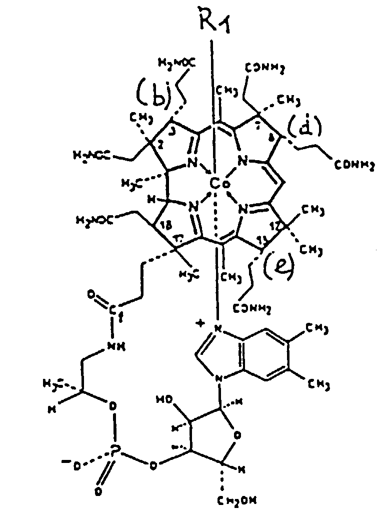 par clivage d'un groupe -CONH₂, R₁ représentant les différents restes qui différencient les diverses cobalamines, R représente un groupement de liaison (espaceur), choisi parmi alkylène, aralkylène et arylène, qui peut contenir un ou plusieurs hétéroatomes, et x est 0 ou 1.

2. Procédé selon la revendication 1, caractérisé en ce que R est -CO-CH₂-(O-CH₂-CH₂-)ₙO-CH₂ où n est 1, 2 ou 3.

3. Procédé de préparation de conjugués de cobalamine de formule (III)
B-CO-NH-(-NH-R-CO-NH-)ₓ-N=GP (III)
où B, R et x possèdent la signification indiquée dans la revendication 1 ou 2 et GP représente le reste d'une protéine contenant des groupes glycosyle qui est liée par le biais d'un reste glycosyle au groupement -NH-N=.

4. Procédé selon la revendication 3, caractérisé en ce que GP représente le reste d'une enzyme de marquage.

5. Utilisation d'un hydrazide d'acide de cobalamine selon la revendication 1 ou 2 pour la préparation de conjugués de cobalamine, en particulier de conjugués cobalamine/glycoprotéine selon la revendication 3.

6. Utilisation d'un conjugué de cobalamine selon la revendication 3 ou 4 pour des méthodes immunologiques d'examen et d'analyse, en particulier dans des immunodosages pour la détermination de la cyanocobalamine.

7. Procédé de préparation de bis-hydrazide d'acide triéthyléneglycoldiacétique (TEDEH), caractérisé en ce que l'on convertit le triéthylèneglycol par réaction avec le bromoacétate de tert.butyle en le triéthylèneglycoldiacétate de bis-tert.butyle, puis on convertit celui-ci en l'acide dicarboxylique libre, on convertit l'acide dicarboxylique libre avec la tert.butyloxycarbonylhydrazine en le bis-(BOC-hydrazide) d'acide triéthylèneglycoldiacétique, et on convertit le produit réactionnel ainsi obtenu en le bis-hydrazide d'acide triéthyléneglycoldiacétique ou en le chlorhydrate correspondant par clivage des restes BOC.

8. Utilisation du bis-hydrazide d'acide triéthylèneglycoldiacétique (TEDEH) pour introduire un groupement espaceur dans des conjugués de cobalamine de formule (III) selon la revendication 3.
